(19) Europäisches Patentamt
European Patent Office
Office européen des brevets

(11) Veröffentlichungsnummer: **0 330 826**
**A2**

# (12) EUROPÄISCHE PATENTANMELDUNG

(21) Anmeldenummer: **89101034.0**

(22) Anmeldetag: **21.01.89**

(51) Int. Cl.[4]: **A61K 31/44 , A61K 31/445 , A61K 31/55 , A61K 31/46**

Ein Antrag gemäss Regel 88 EPÜ auf Berichtigung der Beschreibung liegt vor. Über diesen Antrag wird im Laufe des Verfahrens vor der Prüfungsabteilung eine Entscheidung getroffen werden (Richtlinien für die Prüfung im EPA, A-V, 2.2).

Patentansprüche für folgende Vertragsstaaten: ES + GR.

(30) Priorität: **03.02.88 DE 3803135**

(43) Veröffentlichungstag der Anmeldung: **06.09.89 Patentblatt 89/36**

(84) Benannte Vertragsstaaten: **AT BE CH DE ES FR GB GR IT LI LU NL SE**

(71) Anmelder: **ASTA Pharma Aktiengesellschaft**
**Weismüllerstrasse 45**
**D-6000 Frankfurt am Main 1(DE)**

(72) Erfinder: **Engel, Jürgen, Dr.**
**Erlenweg 3**
**D-8755 Alzenau(DE)**
Erfinder: **Nickel, Bernd, Dr.**
**Alleestrasse 35**
**D-6109 Mühltal(DE)**
Erfinder: **Szelenyi, Istvan, Dr.**
**Händelstrasse 32**
**D-8501 Schwaig(DE)**

(54) **Verwendung von Pyridin-2-ethern beziehungsweise Pyridin-2-thioethern mit einem stickstoffhaltigen cycloaliphatischen Ring als antidepressiv wirkende Arzneimittel.**

(57) Verwendung von Wirkstoffen der Formel

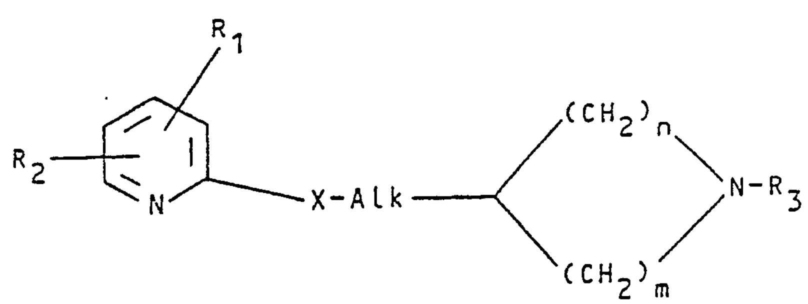

I

worin die Reste $R_1$ und $R_2$ beispielsweise Wasserstoff, Halogenatome oder Trifluormethyl bedeuten, der Rest $R_3$ beispielsweise Wasserstoff, eine $C_1$-$C_6$-Alkylgruppe, eine $C_3$-$C_6$-Alkenylgruppe, eine $C_3$-$C_6$-Alkinylgruppe, eine $C_3$-$C_7$-Cycloalkylgruppe, eine $C_5$-$C_7$-Cycloalkenylgruppe, eine Phenyl-$C_1$-$C_4$-alkylgruppe, eine Carb-$C_1$-$C_6$-alkoxygruppe, eine gegebenenfalls durch einen $C_3$-$C_6$-Cycloalkylrest substituierte $C_2$-$C_6$-Alkanoylgruppe oder eine $C_1$-$C_4$-Alkylgruppe ist, die am selben C-Atom zwei $C_1$-$C_6$ Alkoxygruppen oder eine $C_2$-$C_4$-Alkylendioxygruppe enthält, X Sauerstoff, Schwefel, SO oder $SO_2$ bedeutet, Alk Alkylen mit 0-4 C-Atomen ist und n und m gleich oder verschieden sind und die Zahlen 1-3 annehmen können, wobei n auch 0 sein kann, wenn Alk Alkylen mit mindestens einem Kohlenstoffatom ist und m für diesen Fall die Zahlen 2-8 annimmt und wobei die Gruppierung

EP 0 330 826 A2

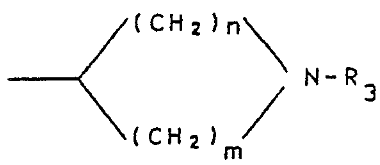

auch den Chinuclidylrest oder den Tropanylrest darstellen kann, deren Pyridin-N-oxiden und deren pharmazeutisch verwendbaren Salzen für die Herstellung von Arzneimitteln zur Bekämpfung von Depressionen.

## Verwendung von Pyridin-2-ethern beziehungsweise Pyridin-2-thioethern mit einem stickstoffhaltigen cycloaliphatischen Ring als antidepressiv wirkende Arzneimittel

Die Europäische Patentanmeldung 149 088 betrifft neue Pyridin-2-ether beziehungsweise Pyridin-2-thioether mit einem stickstoffhaltigem cycloaliphatischen Ring. Für diese Verbindungen wird eine analgetische und blutdrucksenkende Wirkung angegeben.

Es wurde nun gefunden, daß diese Verbindungen zusätzlich eine antidepressive Wirkung besitzen. Für diese antidepressive Wirkung kommen deutlich höhere Dosierungsen in Frage als für die analgetische und blutdrucksendende Wirkung.

Die Erfindung betrifft also eine neue erfinderische Indikation von Verbindungen der Formel I gemäß der Definition von Anspruch 1.

Bei dieser neuen erfindungsgemäßen Wirkung handelt es sich um eine antidepressive Wirkung.

Der Erfindung liegt also die Aufgabe zugrunde, Verbindungen der Formel I gemäß Patentanspruch 1 als antidepressiv wirkende Wirkstoffe zur Verfügung zu stellen und diese Wirkstoffe als antidepressiv wirkende Arzneimittel zu verwenden.

Die folgenden Erläuterungen stellen erfindungswesentliche Angaben dar:

Die in der Formel I vorkommenden Alkylgruppen, Alkenylgruppen, Alkinylgruppen, Alkoxygruppen, Alkanoylaminogruppen oder Alkanoylgruppen können gerade oder verzweigt sein. Dasselbe gilt auch für Alkyl- und Alkoxygruppen, falls diese Bestandteil anderer zusammengesetzter Gruppen sind (zum Beispiel in Form einer Monoalkyl- oder Dialkylaminogruppe, Alkanoylaminogruppe, Alkoxycarbonylaminogruppe, Carbalkoxygruppe, Alkylcarbonylgruppe und ähnlichen Gruppen. Desgleichen kann bei der Bedeutung Phenyl-$C_1$-$C_6$-alkylrest(gruppe) der Alkylteil, falls dieser aus 2-4 C-Atomen besteht, ebenfalls gerade oder verzweigt sein. Bei den Halogenatomen handelt es sich um Chlor, Brom oder Fluor, insbesondere Chlor und Fluor. Falls $R_1$ und/oder $R_2$ eine Aminogruppe bedeutet, die durch Halogenbenzyl substituiert ist, handelt es sich vorzugsweise um Chlorbenzyl oder Fluorbenzyl, wobei Chlor beziehungsweise Fluor sich vorzugsweise in 4-Stellung des Phenylrestes befinden.

Die Alkyl- und Alkoxygruppen als solche oder als Bestandteil von anderen zusammengesetzten Gruppen bestehen insbesondere aus 1-4 C-Atomen, vorzugsweise 1 oder 2 C-Atomen. Die Alkenylgruppen beziehungsweise Alkinylgruppen bestehen vorzugsweise aus 3 oder 4 C-Atomen. Alkanoylgruppen oder Alkanoylaminogruppen bestehen insbesondere aus 2-4, vorzugsweise 2-3 C-Atomen. Der Alkylteil des Phenyl-$C_1$-$C_4$-alkylrestes(gruppe) besteht insbesondere aus 1-3, vorzugsweise 1 oder 2 C-Atomen. Die $C_3$-$C_7$-Cycloalkylgruppe besteht insbesondere aus 5 bis 6 C-Atomen, die $C_5$-$C_7$-Cycloalkenylgruppe insbesondere aus 5 bis 6 C-Atomen. Die $C_2$-$C_4$-Alkylendioxygruppe besteht insbesondere aus 2 bis 3 C-Atomen. Die Gruppe

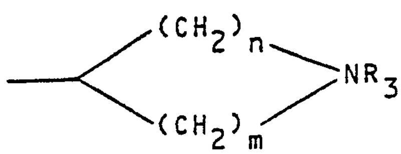

bildet insbesondere einen 5-, 6- oder 7-Ring.

Beispiele hierfür sind: Piperidinring (Piperidyl-(4)-, Piperidyl-(3)- oder Piperidyl-(2)-ring), Homopiperidinring (zum Beispiel Homopiperidyl-(4)-ring), Pyrrolidinring (Pyrrolidyl-(2)- oder Pyrrolidyl-(3)-ring). Als Chinuclidinring kommt vorzugsweise der Chinuclidyl-(3)-rest, als Tropanylring der Tropanyl-(3)-rest in Frage.

X bedeutet vorzugsweise Schwefel.

Besonders wichtig sind solche Verbindungen der Formel I, worin X Schwefel, einer der Reste $R_1$ beziehungsweise $R_2$ Wasserstoff, der gesättigte stickstoffhaltige Ring ein Piperidylrest ist, der direkt mit dem Schwefelatom verbunden ist (Alk = 0 C-Atome, das heißt Alk entfällt) und $R_3$ Wasserstoff, eine $C_3$-$C_6$-Alkenylgruppe (gerade oder verzweigt) oder eine gerade oder verzweigte $C_1$-$C_6$-Alkylgruppe, die am endständigen C-Atom auch 2 $C_1$-$C_4$-Alkoxygruppen oder eine $C_2$-$C_4$-Alkylendioxygruppe enthalten kann, darstellt. Hierbei enthält der Pyridinring vorzugsweise einen Substituenten entsprechend den angegebenen Bedeutungen für $R_1$/$R_2$, vorzugsweise ist dieser Substituent ein Halogenatom (beispielsweise Chlor), welches sich insbesondere in 6-Stellung des Pyridinringes befindet. Falls Alk vorhanden ist, besteht diese Gruppe insbesondere aus 1 oder 2 C-Atomen.

Die erfindungsgemäßen Verbindungen der Formel I zeigen zum Beispiel im Schwimm-Test nach

Porsolt eine gute dosisabhängige antidepressive Wirkung.

Der Schwimm-Text an der Ratte erfolgt in Anlehnung an die Methode von Porsolt (Porsolt, R.D., A. Bertin and M. lalfre, 1977, Behavioral despair in mice, Arch. Intern. Pharmacodyn. Ther. 229, 327): Hierzu werden Ratten einzeln in einen mit Wasser gefüllten Plastikbehälter gesetzt. Die Höhe des Behälters ist 30 cm, die Länge beträgt 35 cm und die Breite 20 cm. Das Wasser hat eine Temperatur von 25° C und die Tiefe ist 20 cm. Die Schwimmaktivitäts- beziehungsweise die Immobilisationsphasen der Tiere werden mit Hilfe eines motor activity meters über 15 Minuten in einem Abstand von 1 Minute (Impulse/Min.) registriert. Im Normalfall (das heißt ohne Antidepressivum) machen die Tiere nur für kurze Zeit Schwimmbewegungen und lassen sich dann treiben. Die Zunahme der Aktivitäts- beziehungsweise Abnahme der Immobilisationsphasen (die Tiere schwimmen nicht, sondern lassen sich treiben) gilt als Maß für die antidepressive Wirkung einer Substanz. Die antidepressive Wirkung wird als Wirkung in % für eine bestimmte Dosis antidepressive Wirksubstanz angegeben bezogen auf eine Kontrollgruppe, die keine Wirkstubstanz bekommen hat.

Beispielsweise wird bei oben genannter Versuchsmethode im Falle der Verbindung 6-Chlor-2- piperidyl-(4)-thio-pyridin (Anpirtolin) bei einer Dosis von 6 mg/kg Körpergewicht Ratte (intraperitoneale Applikation) eine Wirkung von 50 % erhalten.

Die niedrigste, bereits wirksame Dosis in dem oben angegebenen Tierversuch liegt beispielsweise zwischen

5 - 40 mg/kg oral (für Anpirtolin bei 20 mg/kg) und

0,1 - 1 mg/kg intravenös (für Anpirtolin bei 0,5 mg/kg)

Als allgemeiner Dosisbereich für die antidepressive Wirkung (Tierversuch wie oben) kommt beispielsweise in Frage:

5 - 200 mg/kg oral, insbesondere 10 bis 100 mg

0,1 - 20 mg/kg intravenös, insbesondere 1 bis 5 mg

Diese Wirkungsrichtung ist mit der Verbindung des bekannten Arzneimittelwirkstoffs Imipramin vergleichbar, jedoch ist die antidepressive Wirkung der Verbindungen der Formel I stärker (zum Beispiel um den Faktor 5) und länger anhaltend.

Indikationen für die die neue Verwendung der Verbindungen der Formel I in Betracht kommt: Depression jeglicher Art, vor allem solche, wo Stimmungsaufhellung erforderlich ist.

Die pharmazeutischen Zubereitungen für die Anwendung als Antidepressiva enthalten beispielsweise zwischen 1 bis 200 mg, vorzugsweise 10 bis 100 mg, insbesondere 20 bis 50 mg der erfindungsgemäßen aktiven Komponente.

Die Verabreichung kann beispielsweise in Form von Tabletten, Kapseln, Pillen, Dragees, Zäpfchen, Salben, Gelees, Cremes, Puder, Stäubepulver, Aerosolen oder in flüssiger Form erfolgen. Als flüssige Anwendungsformen kommen zum Beispiel in Frage: Ölige oder alkoholische beziehungsweise wässrige Lösungen sowie Suspenionen und Emulsionen. Bevorzugte Anwendungsformen sind Tabletten, die zwischen 20 und 100 mg oder Lösungen, die zwischen 0,1 bis 10 Gewichtsprozent an aktiver Substanz enthalten.

Die Einzeldosis der erfindungsgemäßen aktiven Komponenten kann beispielsweise liegen

a) bei oralen Arzneiformen zwischen 10 und 200 mg

b) bei parenteralen Arzneiformen (zum Beispiel intravenös, intramuskulär) zwischen 1 und 10 mg.

Beispielsweise können 3mal täglich 1 bis 3 Tabletten mit einem Gehalt von 20 bis 100 mg wirksamer Substanz oder zum Beispiel bei intravenöser Injektion 1 bis 3mal täglich eine Ampulle von 1 bis 10 ml Inhalt mit 1 bis 5 mg Substanz empfohlen werden. Bei oraler Verabreichung ist die minimale tägliche Dosis beispielsweise 60 mg; die maximale tägliche Dosis bei oraler Verabreichung soll im allgemeinen nicht über 600 mg liegen.

Die akute Toxizität der erfindungsgemäßen Verbindungen an der Maus (ausgedrückt durch die LD 50 mg/kg; Methode nach Miller und Tainter: Proc. Soc. Exper. Biol. a. Med. 57 (1944) 261) liegt beispielsweise bei oraler Applikation zwischen 70 und 200 mg/kg (beziehungsweise oberhalb 80, vorzugsweise oberhalb 140 mg/kg).

Die Arzneimittel können in der Humanmedizin, der Veterinärmedizin sowie in der Landwirtschaft allein oder im Gemisch mit anderen pharmakologisch aktiven Stoffen verwendet werden.

- (Die angegebenen Dosen sind jeweils bezogen auf die freie Base) -

Pharmakologische beziehungsweise pharmazeutische Angaben

Die erfindungsgemäßen Verbindungen der Formel I gemäß Anspruch 1 sind zur Herstellung pharmazeutischer Zusammensetzungen und Zubereitungen geeignet. Die pharmazeutischen Zusammensetzungen beziehungsweise Arzneimittel enthalten als Wirkstoff einen oder mehrere der erfindungsgemäßen Verbindungen dieser Formel I, gegebenenfalls in Mischung mit anderen pharmakologisch beziehungsweise pharmazeutisch wirksamen Stoffen. Die Herstellung der Arzneimittel erfolgt in bekannter Weise, wobei die bekannten und üblichen pharmazeutischen Hilfsstoffe sowie sonstige übliche Träger und Verdünnungsmittel verwendet werden können.

Als derartige Träger- und Hilfsstoffe kommen zum Beispiel solche Stoffe in Frage, die in folgenden Literaturstellen als Hilfsstoffe für Pharmazie, Kosmetik und angrenzende Gebiete empfohlen beziehungsweise angegeben sind: Ullmanns Encyklopädie der technischen Chemie, Band 4 (1953), Seite 1 bis 39; Journal of Pharmaceutical Sciences, Band 52 (1963), Seite 918 u. ff.; H.v. Czetsch-Lindenwald, Hilfsstoffe für Pharmazie und angrenzende Gebiete; Pharm. Ind., Heft 2, 1961, Seite 72 u. ff.; Dr. H. P. Fiedler, Lexikon der Hilfsstoffe für Pharmazie, Kosmetik und angrenzende Gebiete, Cantor KG. Aulendorf in Württemberg 1971.

Die pharmazeutische und galenische Handhabung der erfindungsgemäßen Verbindungen der Formel I erfolgt nach den üblichen Standardmethoden. Beispielsweise werden Wirkstoffe und Hilfs- beziehungsweise Trägerstoffe durch Rühren oder Homogenisieren (zum Beispiel mittels üblicher Mischgeräte) gut vermischt, wobei im allgemeinen bei Temperaturen zwischen 20 und 80° C, vorzugsweise 20 bis 50° C, insbesondere bei Raumtemperatur gearbeitet wird. Im übrigen wird auf das folgende Standardwerk verwiesen: Sucker, Fuchs, Speiser, Pharmazeutische Technologie, Thieme-Verlag Stuttgart, 1978.

Die erfindungsgemäßen antidepressiv wirkenden Arzneimittel können für orale Applikation bevorzugt 10 bis 200 mg, vorzugsweise 20 bis 100 mg an Wirkstoffen der Formel I, für parenterale Applikation bevorzugt 1 bis 10 mg, beispielsweise 2 bis 5 mg enthalten. Aber auch 6 bis 10 mg pro Zubereitung kommen für die parenterale Anwendung in Frage.

Hierbei kann es sich bei den zuvor angegebenen Mengen an Wirkstoff I um die Menge der einzelnen Verbindung der Formel I gemäß Anspruch 1 handeln, oder es handelt sich um die Gesamtmenge aus mehreren Einzelverbindungen der Formel I.

Beispiel 1

Ampullen mit 2,5 mg Anpirtolinhydrochlorid in 2 ml

2,5 g Anpirtolinhydrochlorid werden zusammen mit 15,8 g Natriumchlorid in 1,8 Liter Wasser für Injektionszwecke kalt gelöst. Die Lösung wird durch Zugabe von Natronlauge 0,1 N auf einen pH-Wert von 7,4 eingestellt. Danach wird die erhaltene Lösung mit Wasser für Injektionszwecke auf 2 Liter aufgefüllt und 20 Minuten lang mit Stickstoff begast. Die Lösung wird anschließend über ein Membranfilter der Porenweite 0,2 µm mit Glasfaservorfilter filtriert und zu 2,2 ml in 2ml-Ampullen abgefüllt, wobei gleichzeitig mit Stickstoff begast wird.

Die Ampullen werden 15 Minuten lang bei 121° C autoklaviert.

2 ml Injektionslösung enthalten 2,5 mg Anpirtolinhydrochlorid.

Beispiel 2

Kapseln mit 20 mg Anpirtolinhydrochlorid

400 g Anpirtolinhydrochlorid werden mit 880 g Lactose und 700 g mikrokristalliner Cellulose sowie anschließend mit 20 g Magnesiumstearat gemischt. Die Mischung wird zu jeweils 100 mg in Hartgelatinekapseln der Größe 3 gefüllt.

Eine Kapsel enthält 20 mg Anpirtolinhydrochlorid.

## Ansprüche

1. Verwendung von Wirkstoffen der Formel

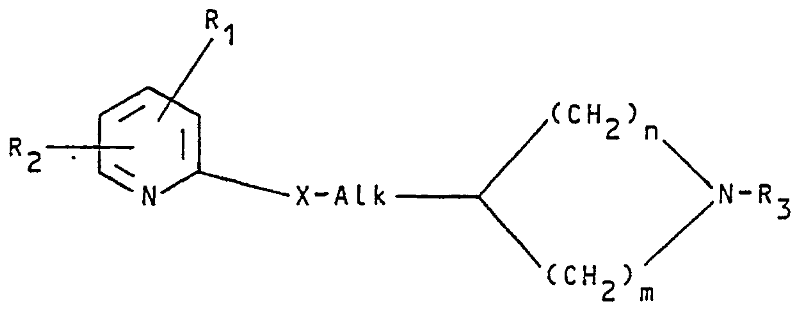

I ,

worin die Reste $R_1$ und $R_2$ gleich oder verschieden sind und Wasserstoff, Halogenatome, eine Trifluormethylgruppe, eine Cyanogruppe, eine Nitrogruppe, eine Aminogruppe, eine Mono-$C_1$-$C_6$-alkylaminogruppe, eine Di-$C_1$-$C_6$-alkylaminogruppe, eine Aminogruppe, die durch einen Halogenbenzylrest oder einen Phenyl-$C_1$-$C_4$-alkylrest substituiert ist, eine $C_1$-$C_6$-Alkanoylaminogruppe, eine $C_1$-$C_6$-Alkoxycarbonylaminogruppe, eine gegebenenfalls durch einen Phenylrest substituierte $C_1$-$C_6$-Alkylgruppe, eine Phenylgruppe, eine Hydroxygruppe, eine $C_1$-$C_6$-Alkoxygruppe, eine Phenoxygruppe, eine Carboxygruppe, eine Carb-$C_1$-$C_6$-alkoxygruppe oder eine gegebenenfalls durch eine oder zwei $C_1$-$C_6$-Alkylgruppen substituierte Carbamoylgruppe bedeuten, der Rest $R_3$ Wasserstoff, eine $C_1$-$C_6$-Alkylgruppe, eine $C_3$-$C_6$-Alkenylgruppe, eine $C_3$-$C_6$-Alkinylgruppe, eine $C_3$-$C_7$-Cycloalkylgruppe, eine $C_5$-$C_7$-Cycloalkenylgruppe, eine Phenyl-$C_1$-$C_4$-alkylgruppe, eine Carb-$C_1$-$C_6$-alkoxygruppe, eine gegebenenfalls durch einen $C_3$-$C_6$-Cycloalkylrest substituierte $C_2$-$C_5$-Alkanoylgruppe oder eine $C_1$-$C_4$-Alkylgruppe ist, die am selben C-Atom zwei $C_1$-$C_6$-Alkoxygruppen oder eine $C_2$-$C_4$-Alkylendioxygruppe enthält oder worin $R_3$ eine $C_1$-$C_6$-Alkylgruppe bedeutet, die ein-oder zweifach durch $C_3$-$C_7$-Cycloalkylgruppen, Hydroxygruppen, $C_1$-$C_6$-Alkoxygruppen, Halogenatome, Sulfogruppen (-$SO_3H$), Aminogruppen, $C_1$-$C_6$-Alkylaminogruppen, Di-$C_1$-$C_6$-alkylaminogruppen, $C_1$-$C_6$-Alkylcarbonylgruppen, $C_3$-C-Cycloalkylcarbonylgruppen, Carb-$C_1$-$C_6$-alkoxygruppen oder Benzoylgruppen substituiert ist, X Sauerstoff, Schwefel, SO oder $SO_2$ bedeutet, Alk Alkylen mit 0-4 C-Atomen ist und n und m gleich oder verschieden sind und die Zahlen 1-3 annehmen können, wobei n auch 0 sein kann, wenn Alk Alkylen mit mindestens einem Kohlenstoffatom ist und m für diesen Fall die Zahlen 2-6 annimmt und wobei die Gruppierung

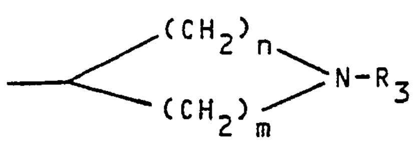

auch den Chinuclidylrest oder den Tropanylrest darstellen kann, deren Pyridin-N-oxiden und deren pharmazeutisch verwendbaren Salzen für die Herstellung von Arzneimitteln zur Bekämpfung von Depressionen.

2. Verwendung von Wirkstoffen als antidepressiv wirkende Arzneimittel gemäß Anspruch 1, worin $R_1$ Wasserstoff, Amino oder $C_2$-$C_6$-Alkanoyl-amino, $R_2$ Chlor, Brom, Fluor, $C_1$-$C_4$-Alkyl oder $C_1$-$C_4$-Alkoxy, X Schwefel und $R_3$ Wasserstoff, $C_3$-$C_6$-Alkenyl oder $C_1$-$C_6$-Alkyl, welches auch ein Halogenatom, eine Methylendioxygruppe oder eine oder zwei Hydroxygruppen enthalten kann, bedeutet, und worin der basisch gesättigte Ring

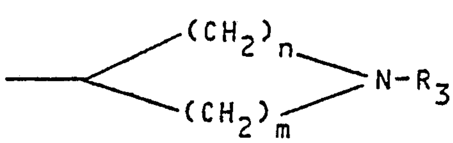

einen Pyrrolidylrest, einen Piperidylrest oder einen Homopiperidylrest darstellt, welcher jeweils am N-Atom den Rest $R_3$ enthält.

3. Verwendung von Wirkstoffen als antidepressiv wirkende Arzneimittel gemäß Anspruch 1, worin $R_1$ Wasserstoff, $R_2$ Chlor, Brom oder Fluor, X Schwefel und $R_3$ Wasserstoff oder eine $C_1$-$C_6$-Alkylgruppe bedeutet, und der basische Ring

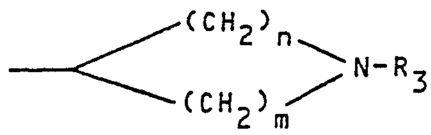

einen Pyrrolidylrest, einen Piperidylrest oder einen Homopiperidylrest darstellt, welcher jeweils am N-Atom den Rest $R_3$ enthält.

4. Antidepressiv wirkendes Arzneimittel nach einem oder mehreren der vorangegangenen Ansprüche, dadurch gekennzeichnet,
daß es den Wirkstoff beziehungsweise die Wirkstoffe gemäß der Formel I zusammen mit üblichen pharmazeutischen Trägern, Hilfsstoffen und/oder Verdünnungsmitteln enthält.

5. Antidepressiv wirkendes Arzneimittel nach einem oder mehreren der vorangegangenen Ansprüche, dadurch gekennzeichnet,
daß der Wirkstoff gemäß der Formel I in einer Menge von 1 - 200 mg vorliegt und gegebenenfalls weitere übliche pharmazeutische Trägerstoffe, Hilfsstoffe und/oder Verdünnungsmittel vorhanden sind.

6. Antidepressiv wirkende Arzneimittel nach Anspruch 4 oder 5, gekennzeichnet durch einen Gehalt von mindestens einer Verbindung der Formel I gemäß Anspruch 1, wobei der Gesamtwirkstoffgehalt an Wirkstoff für orale Applikation mindestens 10 mg und für parenterale Applikation mindestens 1 mg beträgt.

7. Verfahren zur Herstellung eines antidepressiv wirkenden Mittels, dadurch gekennzeichnet, daß man mindestens eine Verbindung der Formel I, worin die Symbole $R_1$, $R_2$, $R_3$, Alk, n und m die angegebenen Bedeutungen haben oder deren pharmazeutisch verwendbare Salze, zusammen mit üblichen Träger- und/oder Verdünnungsbeziehungsweise Hilfsstoffen bei Temperaturen zwischen 0 und 120° C, vorzugsweise 20 und 80° C vermischt beziehungsweise homogenisiert und gegebenenfalls die so erhaltene Mischung zur Herstellung von Zubereitungen, die in der Dosierungseinheit 10 bis 200 mg Wirkstoff für orale Applikation und 1 bis 20 mg Wirkstoff für parenterale Applikation enthalten, in Hohlzellen entsprechender Größe ausgießt oder in Kapseln entsprechender Größe abfüllt oder granuliert und dann gegebenenfalls unter Zusatz von weiteren üblichen Hilfsstoffen zu Tabletten verpreßt, oder in Kapseln abfüllt.

8. Verfahren zur Herstellung eines antidepressiv wirkenden Mittels, dadurch gekennzeichnet, daß man mindestens eine Verbindung der Formel I oder deren Salz mit einer physiologisch unbedenklichen Säure mit einem oder mehreren der folgenden Stoffe: Stärke, Cellulose, Lactose, Formalin-Casein, modifizierte Stärke, Magnesiumstearat, Calciumhydrogenphosphat, hochdisperse Kieselsäure, Talkum, Phenoxyethanol vermischt, die erhaltene Mischung gegebenenfalls mit einer wässrigen Lösung, die als Bestandteil mindestens Gelatine, Stärke, Polyvinylpyrrolidon, Vinylpyrrolidon-Vinylacetat-Copolymerisat und/oder Polyoxyethylensorbitanmonooleat enthält, granuliert, das Granulat gegebenenfalls mit einem oder mehreren der oben genannten Hilfsstoffe homogenisiert, und diese Mischung zu Tabletten verpreßt oder in Kapseln abfüllt, wobei die Tabletten oder Kapseln in der Dosierungseinheit jeweils 10 bis 200 mg Wirkstoff enthalten.

9. Verfahren zur Herstellung eines antidepressiv wirkenden Mittels, dadurch gekennzeichnet, daß man mindestens eine Verbindung der Formel I oder deren Salz mit einer physiologisch unbedenklichen Säure mit Sojalecithin sowie gegebenenfalls 0,1 - 0,5 Gewichtsteilen Phenoxyethanol (bezogen auf einen Gewichtsteil Wirkstoff I) bei Temperaturen zwischen 33 - 37° C in geschmolzenem Hartfett suspendiert und homogenisiert und anschließend die Mischung in Hohlzellen ausgießt, wobei die Dosierungseinheit 10 bis 200 mg Wirkstoff enthält.

10.Verfahren zur Herstellung eines antidepressiv wirkenden Mittels, dadurch gekennzeichnet, daß man mindestens eine Verbindung der Formel I oder deren Salz mit einer physiologisch unbedenklichen Säure bei einer Temperatur zwischen 50 bis 120° C, gegebenenfalls in Gegenwart eines oder mehrerer Emulgatoren und/oder 0,1 - 0,5 Gewichtsteilen Phenoxyethanol (bezogen auf 1 Gewichtsteil Wirkstoff I) mit mindestens einem der folgenden Stoffe homogenisiert: Paraffin, Vaseline, aliphatischer Alkohol mit 12 bis 25 C-Atomen, Sorbitanmonopalmitat, aliphatische Monocarbonsäure mit 15 bis 20 C-Atomen, Polyoxyethylenpolyolfettsäureester, und gegebenenfalls unter Zusatz eines mehrwertigen niederen aliphatischen Alkohols emulgiert.

11. Verfahren zur Herstellung eines antidepressiv wirkenden Mittels (Lösung), dadurch gekennzeichnet, daß man mindestens eine Verbindung der Formel I oder deren Salz mit einer physiologisch unbedenklichen Säure gegebenenfalls in Gegenwart von 0,1 - 0,5 Gewichtsteilen Phenoxyethanol (bezogen auf einen

Gewichtsteil Wirkstoff I) und/oder Natriumchlorid sowie gegebenenfalls in Anwesenheit eines Emulgators, bei Temperaturen zwischen 30 - 100 °C in Wasser, physiologisch unbedenklichen Alkoholen oder Ölen oder Mischungen hiervon auflöst, und gegebenenfalls die so erhaltene Lösung mit soviel Wasser, Alkohol oder Pflanzenöl auffüllt, daß die Endlösung oder Endsuspension 0,1 - 10 Gewichtsprozent an Wirkstoff I enthält.

Patentansprüche für folgende Vertragsstaaten: SGR, ES

1. Verwendung von Wirkstoffen der Formel

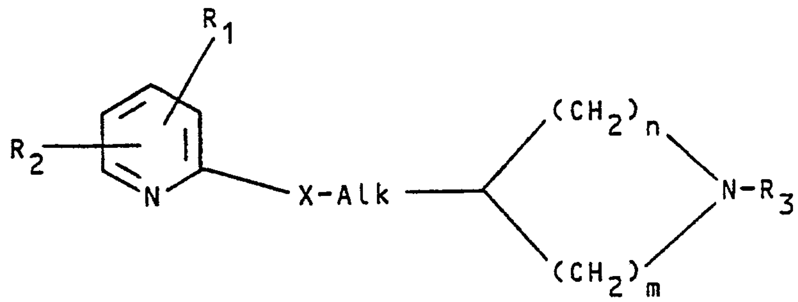

I

worin die Reste $R_1$ und $R_2$ gleich oder verschieden sind und Wasserstoff, Halogenatome, eine Trifluormethylgruppe, eine Cyanogruppe, eine Nitrogruppe, eine Aminogruppe, eine Mono-$C_1$-$C_6$-alkylaminogruppe, eine Di-$C_1$-$C_6$-alkylaminogruppe, eine Aminogruppe, die durch einen Halogenbenzylrest oder einen Phenyl-$C_1$-$C_4$-alkylrest substituiert ist, eine $C_1$-$C_6$-Alkanoylaminogruppe, eine $C_1$-$C_6$-Alkoxycarbonylaminogruppe, eine gegebenenfalls durch einen Phenylrest substituierte $C_1$-$C_6$-Alkylgruppe, eine Phenylgruppe, eine Hydroxygruppe, eine $C_1$-$C_6$-Alkoxygruppe, eine Phenoxygruppe, eine Carboxygruppe, eine Carb-$C_1$-$C_6$-alkoxygruppe oder eine gegebenenfalls durch eine oder zwei $C_1$-$C_6$-Alkylgruppen substituierte Carbamoylgruppe bedeuten, der Rest $R_3$ Wasserstoff, eine $C_1$-$C_6$-Alkylgruppe, eine $C_3$-$C_6$-Alkenylgruppe, eine $C_3$-$C_6$-Alkinylgruppe, eine $C_3$-$C_7$-Cycloalkylgruppe, eine $C_5$-$C_7$-Cycloalkenylgruppe, eine Phenyl-$C_1$-$C_4$-alkylgruppe, eine Carb-$C_1$-$C_6$-alkoxygruppe, eine gegebenenfalls durch einen $C_3$-$C_6$-Cycloalkylrest substituierte $C_2$-$C_6$-Alkanoylgruppe oder eine $C_1$-$C_4$-Alkylgruppe ist, die am selben C-Atom zwei $C_1$-$C_6$-Alkoxygruppen oder eine $C_2$-$C_4$-Alkylendioxygruppe enthält oder worin $R_3$ eine $C_1$-$C_6$-Alkylgruppe bedeutet, die ein-oder zweifach durch $C_3$-$C_7$-Cycloalkylgruppen, Hydroxygruppen, $C_1$-$C_6$-Alkoxygruppen, Halogenatome, Sulfogruppen ($-SO_3H$), Aminogruppen, $C_1$-$C_6$-Alkyl-aminogruppen, Di-$C_1$-$C_6$-alkylaminogruppen, $C_1$-$C_6$-Alkylcarbonylgruppen, $C_3$-$C_7$-Cycloalkylcarbonylgruppen, Carb-$C_1$-$C_6$-alkoxygruppen oder Benzoylgruppen substituiert ist, X Sauerstoff, Schwefel, SO oder $SO_2$ bedeutet, Alk Alkylen mit 0-4 C-Atomen ist und n und m gleich oder verschieden sind und die Zahlen 1-3 annehmen können, wobei n auch 0 sein kann, wenn Alk Alkylen mit mindestens einem Kohlenstoffatom ist und m für diesen Fall die Zahlen 2-6 annimmt und wobei die Gruppierung

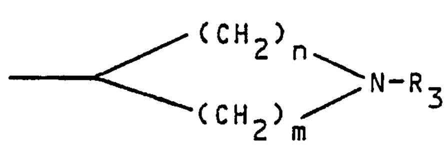

auch den Chinuclidylrest oder den Tropanylrest darstellen kann, deren Pyridin-N-oxiden und deren pharmazeutisch verwendbaren Salzen für die Herstellung von Arzneimitteln zur Bekämpfung von Depressionen.

2. Verfahren zur Herstellung eines antidepressiv wirkenden Mittels , dadurch gekennzeichnet, daß man mindestens eine Verbindung der Formel I

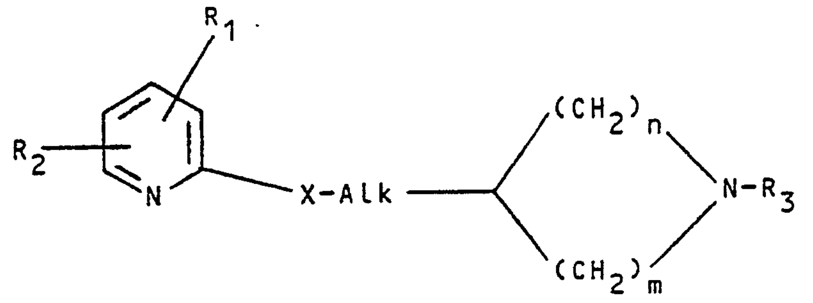

I

worin die Reste $R_1$ und $R_2$ gleich oder verschieden sind und Wasserstoff, Halogenatome, eine Trifluormethylgruppe, eine Cyanogruppe, eine Nitrogruppe, eine Aminogruppe, eine Mono-$C_1$-$C_6$-alkylaminogruppe, eine Di-$C_1$-$C_6$-alkylaminogruppe, eine Aminogruppe, die durch einen Halogenbenzylrest oder einen Phenyl-$C_1$-$C_4$-alkylrest substituiert ist, eine $C_1$-$C_6$-Alkanoylaminogruppe, eine $C_1$-$C_6$-Alkoxycarbonylaminogruppe, eine gegebenenfalls durch einen Phenylrest substituierte $C_1$-$C_6$-Alkylgruppe, eine Phenylgruppe, eine Hydroxygruppe, eine $C_1$-$C_6$-Alkoxygruppe, eine Phenoxygruppe, eine Carboxygruppe, eine Carb-$C_1$-$C_6$-alkoxygruppe oder eine gegebenenfalls durch eine oder zwei $C_1$-$C_6$-Alkylgruppen substituierte Carbamoylgruppe bedeuten, der Rest $R_3$ Wasserstoff, eine $C_1$-$C_6$-Alkylgruppe, eine $C_3$-$C_6$-Alkenylgruppe, eine $C_3$-$C_6$-Alkinylgruppe, eine $C_3$-$C_7$-Cycloalkylgruppe, eine $C_5$-$C_7$-Cycloalkenylgruppe, eine Phenyl-$C_1$-$C_4$-alkylgruppe, eine Carb-$C_1$-$C_6$-alkoxygruppe, eine gegebenenfalls durch einen $C_3$-$C_6$-Cycloalkylrest substituierte $C_2$-$C_6$-Alkanoylgruppe oder eine $C_1$-$C_4$-Alkylgruppe ist, die am selben C-Atom zwei $C_1$-$C_6$-Alkoxygruppen oder eine $C_2$-$C_4$-Alkylendioxygruppe enthält oder worin $R_3$ eine $C_1$-$C_6$-Alkylgruppe bedeutet, die ein-oder zweifach durch $C_3$-$C_7$-Cycloalkylgruppen, Hydroxygruppen, $C_1$-$C_6$-Alkoxygruppen, Halogenatome, Sulfogruppen ($-SO_3H$), Aminogruppen, $C_1$-$C_6$-Alkylaminogruppen, Di-$C_1$-$C_6$-alkylaminogruppen, $C_1$-$C_6$-Alkylcarbonylgruppen, $C_3$-$C_7$-Cycloalkylcarbonylgruppen, Carb-$C_1$-$C_6$-alkoxygruppen oder Benzoylgruppen substituiert ist, X Sauerstoff, Schwefel, SO oder $SO_2$ bedeutet, Alk Alkylen mit 0-4 C-Atomen ist und n und m gleich oder verschieden sind und die Zahlen 1-3 annehmen können, wobei n auch 0 sein kann, wenn Alk Alkylen mit mindestens einem Kohlenstoffatom ist und m für diesen Fall die Zahlen 2-6 annimmt und wobei die Gruppierung

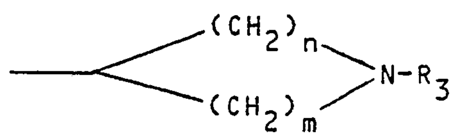

auch den Chinuclidylrest oder den Tropanylrest darstellen kann, deren Pyridin-N-oxiden oder deren pharmazeutisch verwendbare Salze, zusammen mit üblichen Träger- und/oder Verdünnungsbeziehungsweise Hilfsstoffen bei Temperaturen zwischen 0 und 120°C, vorzugsweise 20 und 80°C vermischt beziehungsweise homogenisiert und gegebenenfalls die so erhaltene Mischung zur Herstellung von Zubereitungen, die in der Dosierungseinheit 10 bis 200 mg Wirkstoff für orale Applikation und 1 bis 20 mg Wirkstoff für parenterale Applikation enthalten, in Hohlzellen entsprechender Größe ausgießt oder in Kapseln entsprechender Größe abfüllt oder granuliert und dann gegebenenfalls unter Zusatz von weiteren üblichen Hilfsstoffen zu Tabletten verpreßt, oder in Kapseln abfüllt.

3. Verfahren zur Herstellung eines antidepressiv wirkenden Mittels, dadurch gekennzeichnet, daß man mindestens eine Verbindung der Formel I oder deren Salz mit einer physiologisch unbedenklichen Säure mit einem oder mehreren der folgenden Stoffe: Stärke, Cellulose, Lactose, Formalin-Casein, modifizierte Stärke, Magnesiumstearat, Calciumhydrogenphosphat, hochdisperse Kieselsäure, Talkum, Phenoxyethanol vermischt, die erhaltene Mischung gegebenenfalls mit einer wässrigen Lösung, die als Bestandteil mindestens Gelatine, Stärke, Polyvinylpyrrolidon, Vinylpyrrolidon-Vinylacetat-Copolymerisat und/oder Polyoxyethylensorbitanmonooleat enthält, granuliert, das Granulat gegebenenfalls mit einem oder mehreren der oben genannten Hilfsstoffe homogenisiert, und diese Mischung zu Tabletten verpreßt oder in Kapseln abfüllt, wobei die Tabletten oder Kapseln in der Dosierungseinheit jeweils 10 bis 200 mg Wirkstoff enthalten.

4. Verfahren zur Herstellung eines antidepressiv wirkenden Mittels, dadurch gekennzeichnet, daß man mindestens eine Verbindung der Formel I oder deren Salz mit einer physiologisch unbedenklichen Säure mit Sojalecithin sowie gegebenenfalls 0,1 - 0,5 Gewichtsteilen Phenoxyethanol (bezogen auf einen Gewichtsteil Wirkstoff I) bei Temperaturen zwischen 33 - 37°C in geschmolzenem Hartfett suspendiert und

homogenisiert und anschließend die Mischung in Hohlzellen ausgießt, wobei die Dosierungseinheit 10 bis 200 mg Wirkstoff enthält. 5. Verfahren zur Herstellung eines antidepressiv wirkenden Mittels, dadurch gekennzeichnet, daß man mindestens eine Verbindung der Formel I oder deren Salz mit einer physiologisch unbedenklichen Säure bei einer Temperatur zwischen 50 bis 120° C, gegebenenfalls in Gegenwart eines oder mehrerer Emulgatoren und/oder 0,1 - 0,5 Gewichtsteilen Phenoxyethanol (bezogen auf 1 Gewichtsteil Wirkstoff I) mit mindestens einem der folgenden Stoffe homogenisiert: Paraffin, Vaseline, aliphatischer Alkohol mit 12 bis 25 C-Atomen, Sorbitanmonopalmitat, aliphatische Monocarbonsäure mit 15 bis 20 C-Atomen, Polyoxyethylenpolyolfettsäureester, und gegebenenfalls unter Zusatz eines mehrwertigen niederen aliphatischen Alkohols emulgiert.

6. Verfahren zur Herstellung eines antidepressiv wirkenden Mittels (Lösung), dadurch gekennzeichnet, daß man mindestens eine Verbindung der Formel I oder deren Salz mit einer physiologisch unbedenklichen Säure gegebenenfalls in Gegenwart von 0,1 - 0,5 Gewichtsteilen Phenoxyethanol (bezogen auf einen Gewichtsteil Wirkstoff I) und/oder Natriumchlorid sowie gegebenenfalls in Anwesenheit eines Emulgators, bei Temperaturen zwischen 30 - 100° C in Wasser, physiologisch unbedenklichen Alkoholen oder Ölen oder Mischungen hiervon auflöst, und gegebenenfalls die so erhaltene Lösung mit soviel Wasser, Alkohol oder Pflanzenöl auffüllt, daß die Endlösung oder Endsuspension 0,1 - 10 Gewichtsprozent an Wirkstoff I enthält.